Europäisches Patentamt

(19) European Patent Office

Office européen des brevets

(11) Publication number : **0 430 914 A1**

# (12) EUROPEAN PATENT APPLICATION

(21) Application number : 90850381.6

(22) Date of filing : 21.11.90

(51) Int. Cl.⁵ : **G01N 33/68**, G01N 33/564, C07K 13/00

(30) Priority : 28.11.89 SE 8904021

(43) Date of publication of application :
05.06.91 Bulletin 91/23

(84) Designated Contracting States :
AT BE CH DE FR GB IT LI NL SE

(71) Applicant : BIOCARB AB
S-223 70 Lund (SE)

(72) Inventor : Saxena, Ramesh
Kämnärsvägen 8/139
S-222 45 Lund (SE)
Inventor : Bygren, Per
Ritaregränd 18
S-222 47 Lund (SE)
Inventor : Wieslander, Jörgen
Östervängsvägen 20
S-223 60 Lund (SE)

(74) Representative : Burman, Tore et al
AWAPATENT/Bergling & Sundbergh,
Sveavägen 13-15, Box 7645
S-103 94 Stockholm (SE)

(54) Diagnostic process.

(57) A diagnostic process for the detection of renal disorders in mammals including man, comprising the steps :

a) obtaining a test specimen from the mammal subject to diagnosis ;

b) reacting entactin or an active fragment thereof with said specimen ; and

c) registering whether interaction occurs between the entactin and said specimen indicating the presence or absence of said disorder ; and

a diagnostic composition containing entactin or an active fragment thereof in combination with a diagnostically acceptable carrier.

Fig.1

EP 0 430 914 A1

## DIAGNOSTIC PROCESS

The present invention relates to a diagnostic process for the detection of renal disorders in mammals including man.

Glomerular basement membrane (GBM) is involved in a variety of primary and secondary glomerulonephritis which constitute an important group of renal diseases in man. It is now well established that the majority of glomerulonephritis have immunological origin. The two major immunologic mechanisms causing glomerular injury are immune complex disease and anti-GBM disease. Although constituting only a minority of the glomerular diseases, anti-GBM nephritis is an important clinical entity. Most of these patients have severe progressive renal disease with an extremely high mortality if left untreated. The only well known prototype of anti-GBM nephritis is Goodpasture Syndrome consisting of nephritis, with or without pulmonary haemorrhage and linear deposit of IgG on GBM on direct immunofluorescence. The antigen involved in Goodpasture Syndrome is located on the M2 peptide which is the non-collagenous globular domain (NC1) of the $\alpha$ 3 chain of collagen IV. A few earlier studies have hinted a possible role of other GBM antigens (Non Goodpasture GBM antigens) in the pathogenesis of glomerulonephritis in man. They suggested that anti-GBM antibodies occur more frequently than is generally thought and may play a role in many more types of glomerulonephritis than in the only well defined form of anti-GBM nephritis. Kefalides and coworkers reported the presence of anti-laminin and anti-collagen IV antibodies in patients with post streptococcal glomerulonephritis. Recently, it has been shown that circulating IgA antibodies in complexes with fibronectin bind to the collagen IV in IgA nephropathy. Nevertheless, the involvement of autoantibodies to glomerular antigens, other than the Goodpasture antigen, in the pathogenesis of autoimmune nephritis in man still remains unclear. On the other hand, several glomerular antigens have been described in experimental glomerulonephritis in animals ; such as 330 K and 90 K glycoproteins (gp330 and gp90) in Heymann nephritis, laminin and collagen IV in mercuric chloride induced nephritis in rats and laminin in murine model of graft versus host (GvH) disease.

In accordance with the present invention it has been surprisingly found, that a known protein, named entactin or nidogen (ref. 1,2) interacts with antibodies found in patients having various types of glomerulonephritis. This finding thus results in possibility of using such protein in the diagnosis of autoimmune glomerulonephritis in mammals including man.

Accordingly, the present invention provides for a diagnostic process for the detection of renal disorders in mammals including man, said process comprising the steps :

a) obtaining a test specimen from the mammal subject to diagnosis ;

b) reacting entactin or an active fragment thereof with said specimen ; and

c) registering whether interaction occurs between the entactin and said specimen indicating the presence or absence of said disorder.

As a specimen bodyfluids, such as blood, urine, saliva etc. can be used. It is preferred to use as a test specimen blood samples.

Recordal of the antibody-antigen interaction is suitably performed using ELISA, RIA, hemagglutination-inhibition or the like.

The present invention enables diagnosis constituted by the detection of proliferative glomerulonephritis.

The invention also includes within its scope diagnostic compositions containing entactin (nidogen) or an active fragment thereof in combination with a diagnostically acceptable carrier or diluent.

The invention will be further described in the following by non-limiting examples with reference to the appended drawings, wherein :

Fig. 1 shows a diagram on DEAE-cellulose chromatography of the 6M guanidine-HCl extract of bovine GBM ;

Fig. 2 shows a diagram on sephacryl S-300 gel filtration chromatography of the 150 K protein ;

Fig. 3 shows a diagram on purification of the 150 K protein on a Mono-S FPLC column ;

Fig. 4 shows a chromatogram on SDS-PAGE of the pools containing 150 K protein ;

Fig. 5 shows a diagram illustrating the specificity of the antibodies to the crude guanidine extract of GBM ;

Fig. 6 shows an immunoblotting nitrocellulose paper illustrating the specificity of the antibodies to 6 M guanidine-HCl extract of bovine GBM in an immunblotting experiment ;

Fig. 7 shows a diagram on the precipitation of the 150 K protein by two different antibodies in a radioimmunoassay ;

Fig. 8 shows a diagram on the activity of the 150 K protein to different proteins on a sandwich ELISA ;

Fig. 9 shows diagrams on the inhibition of anti-150 K antibodies by nidogen ;

Fig. 10 is a diagram showing the sensitivity of ELISA for detection of anti-entactin antibodies ;

Fig. 11 shows a diagram on anti-entactin antibodies in patients with glomerulonephritis who have high IgG/IgM/IgA antibodies to the crude guanidine extract of GBM ; and

Fig. 12 is a diagram on the correlation between the presence of anti-entactin and anti-gal $\alpha$ 1,3 gal antibodies.

In the present disclosure the following abbreviations are used :

**cpm**, counts per minute ; **DEAE**, diethylaminoethyl ; **EDTA**, ethylene diamino tetraacetic acid ; **ELISA**, enzyme linked immunosorbant assay ; **FPLC**, fast protein, polypeptide and polynucleotide liquid chromatography ; **GBM**, glomerular basement membrane ; **GvH**, graft versus host ; **HSA**, human serum albumin ; **PBS-BSA**, phosphate buffered saline-bovine serum albumin ; **RIA**, radioimmunoassay ; **SDS-PAGE**, sodium dodecyl sulphate-polyacrylamide gel electrophoresis ; **SLE**, systemic lupus erythematosus.

## EXAMPLE 1

**Preparation of a 150 K protein from GBM and identifcation as entactin/nidogen**

1). *Preparation of GBM :* Bovine GBM was prepared as described earlier. The glomeruli were isolated from bovine kidneys by sieving procedures as described elsewhere (3) in presence of the following protease inhibitors to inhibit endogenous proteolysis : 4 mM N-ethyl-maleimide, 1 mM phenylmetanosulphonyl fluoride, 5 mM benzamidine-HCl, 25 mM 6-aminohexanoic acid and 10 mM EDTA. Isolated glomeruli were sonicated to remove the cellular debris.

2). *Guanidine extraction of GBM :* GBM was briefly homogenised in ten volumes of 6 M guanidine hydrochloride, 0.05 M Tris-HCl, pH 7.5, containing protease inhibitors as above. The extraction was performed overnight at 37°C with continuous stirring. The extract was clarified by centrifugation at 10,000 x g for 60 minutes and the extraction procedure was repeated again with the precipitate. The two extracts were then pooled.

3). *Chromatographic procedures :* 150 ml of the guanidine extract was dialysed against 3 changes of 10 volumes of 6 M urea, 0.05 M Tris-HCl, pH 8.4, containing protease inhibitors : 2.5 mM EDTA, 0.5 mM N-ethyl maleimide and 0.5 mM phenylmetanosulphonyl fluoride and passed over a DEAE Sephacel column (Pharmacia, Uppsala, Sweden) equilibrated with the same buffer. The bound proteins were eluted by a linear salt gradient (0-0.75 M NaCl) and the fractions were analysed by SDS-PAGE on 6-25% gradient polyacrylamide slab gels (4) and by ELISA as described below. Fractions containing 150 K protein were concentrated to 15 ml by YM10 filter (Amicon, Danvers, USA) and applied to a Sephacryl S-300 gel filtration column (Pharmacia, Uppsala, Sweden) equilibrated with 6 M guanidine-HCl, 0.05 M Tris-HCl, pH 7.5. The fractions were analysed, as above, by ELISA and SDS-PAGE after precipitation with 10 volumes of 95% ethanol. The fractions containing 150 K protein were pooled and concentrated to 2 ml, dialysed against 3 changes of 6 M urea, 0.02 M sodium acetate, pH 4.8 containing protease inhibitors and injected to a Mono S FPLC column (Pharmacia, Uppsala, Sweden) equilibrated with the same buffer. The bound proteins were eluted by a linear salt gradient (0-1 M NaCl) and fractions analysed by SDS-PAGE and ELISA. The fractions containing 150 K protein were rechromatographed, if necessary on a Sephacryl S-300 column.

4) *Immunological methods :* Fractions after each chromatographic step were analysed by ELISA (11) as follows :

Each fraction was coated on to one well of a microtiter plate (NUNC ; Roskilde, Denmark) in a dilution of 1 in 20 in 0.05 M sodium carbonate, pH 9.5 and kept overnight at room temperature. The plates were washed 3 times with 0.15 M NaCl, 0.05% Tween 20 and rabbit antiserum raised against the 150 K protein from GBM, diluted 1 in 1000 in PBS-BSA (0.05 M phosphate, 0.15 M NaCl, 0.05% Na azide, pH 7.4, 0.05% Tween 20, 2 mg BSA/ml) added to each well. After incubating for one hour the plates were again washed and anti-rabbit IgG-alkaline phosphatase conjugate (Orion diagnostics, Esbo, Finland), diluted 1 in 1000 in PBS-BSA added. The plates were again incubated for one hour at room temperature, washed and substrate (p-nitrophenyl phosphate, 1 mg/ml in 1 M diethanolamine, pH 9.8 ; 200 µl in each well, Sigma, St. Louis, USA) added. The absorbance was read in a Multiscan (Flow Laboratories, Lugano, Switzerland) microtiter plate reader. Furthermore, the isolated 150 K protein was coated to microtiter plates and ELISA performed as above using 4 different first antibodies (Rabbit anti-150 K polyclonal, rabbit anti-nidogen polyclonal, and mouse anti-nidogen monoclonal $\alpha$ and $\alpha'$ antibodies ; the last three were a gift from Dr. Ralph Butkowski, University of Minnesota, Minneapolis, USA, University of Minnesota, Minneapolis, USA) and anti-rabbit and anti-mouse second antibodies respectively. Next, an inhibition ELISA was performed as follows : Serial dilutions of nidogen (a kind gift of Dr. Ralph Butkowski) and Goodpasture antigens were mixed overnight with a fixed dilution of rabbit anti-150 K antibodies and a representative serum (serum from a patient with glomerulonephritis having antibodies to 150 K protein) and transferred to an ELISA plate coated with the 150 K protein and ELISA performed as described above.

The isolated 150 K was, further, radioiodinated with $^{125}$I by lactoperoxidase method (5). 50 µl of this radiolabeled 150 K (containing approximately 5000 cpm) was mixed with 50 μl of two different antibodies [anti-150 K and anti-nidogen (a kind gift of Dr. Rupert Timpl, Max Plank Institute, Munich, FRG) antibodies] in a serial dilution of PBS-BSA. To this 50 μl of second antibody (anti-rabbit IgG) was added and RIA performed using standard procedures (5).

5). *Amino acid analysis* : was kindly performed by Dr. Mats Paulsson at Max Plank Institute, Munich, FRG.

## EXAMPLE 2

### Analysis of anti-entactin antibodies in patient sera

*1) sera* : Eighty nine sera from patients with renal diseases showing IgG/IgM/IgA antibodies to the crude guanidine extract of GBM, 18 sera from patients with Goodpasture syndrome and 45 normal sera obtained from the blood bank were analysed for the antibodies against entactin that we isolated from the bovine GBM.

*2). Antigens and anti-sera* :

i). The following antigens were used for this study :

a) Entactin as obtained in intact form after various chromatographic steps described above.

b). Gal α 1,3 Gal-HSA (BioCarb AB, Lund, Sweden).

c).Crude 6 M guanidine-HCl extract of bovine GBM

ii). Rabbit anti-150 K antiserum was used as a reference serum.

*3)Immunological methods* :

a) *ELISA* : Antibodies to entactin in sera from various patients, normal sera and the reference serum were tested by sandwich ELISA as follows : Microtiter plates were coated with entactin (145 ng/ml in 0.05 M sodium carbonate, pH 9.5 ; 200 μl in each well) and incubated overnight at room temperature. Sera (200 μl) diluted 1 in 20 in PBS-BSA were added to each well and incubated for one hour at room temperature. The plates were washed 3 times with 0.9% NaCl, 0.05% Tween 20 and the conjugate (200 μl), antihuman IgG/IgM/IgA-alkaline phosphatase and anti-rabbit IgG-alkaline phosphatase (For the reference serum), diluted 1 in 1000 in PBS-BSA added. The plates were incubated for one hour, again washed and the substrate, p-nitrophenyl phosphate added. The absorbance was read at zero time and after 30 minutes in a Multiscan microtiter plate reader. Next, the sera in various dilutions were run using the same protocol. The same procedure was repeated with microtiter plates coated with crude guanidine extract of bovine GBM and Gal α 1,3 Gal-HSA respectively. Furthermore, a fixed dilution of an anti-entactin positive serum was mixed overnight with serial dilutions of entactin and NC1 respectively, and transferred to a microtiter plate coated with crude 6 M guanidine-HCl extract of bovine GBM the next day and ELISA perfomed as described above.

In order to show that the antibodies to entactin detected in various sera are not the antibodies against Gal α 1,3 Gal, a study utilizing competitive ELISA was performed. Two sera, one positive for both entactin and Gal α 1,3 Gal antibodies and the other positive for Gal α 1,3 Gal antibodies only were selected. Various dilutions of these sera were mixed with a fixed dilution of entactin and Gal α 1,3 Gal and allowed to equilibrate overnight at room temperature. Next day they were transferred to the microtiter plates already coated with entactin and Gal α 1,3 Gal and the ELISA was repeated as described above.

b). *Immunoblotting studies* : Western blots were performed according to Burnett (6). Crude guanidine extract of bovine GBM and the 150 K were electrophoresed on polyacrylamide gel and the proteins were transferred to nitrocellulose papers using Transblot SD semi dry transfer cell (Bio Rad Laboratories, CA, USA). After blocking the unbound sites with 5% BSA-PBS the nitrocellulose papers were incubated for 2 hours with the following sera : One serum containing anti-150 K antibodies (serum 1), one negative serum (serum 2) and the reference serum. Additionally, one set of nitrocellulose papers were incubated with the serum 1 already mixed with the 150 K protein. The papers were then washed 3 times with 0.1% BSA-PBS and incubated with the conjugates [Goat anti-human IgG colloid gold and goat anti-rabbit IgG-colloid gold (AuroProbe BLplus, Janssen Biotech, Olen, Belgium)] for 2 hours followed by silver enhancement for 15 minutes using intenSE BL silver enhancement system (Janssen Biotech, Olen, Belgium).

## EXAMPLE 3

### Isolation of 150 K Protein from the Guanidine Extract of GBM :

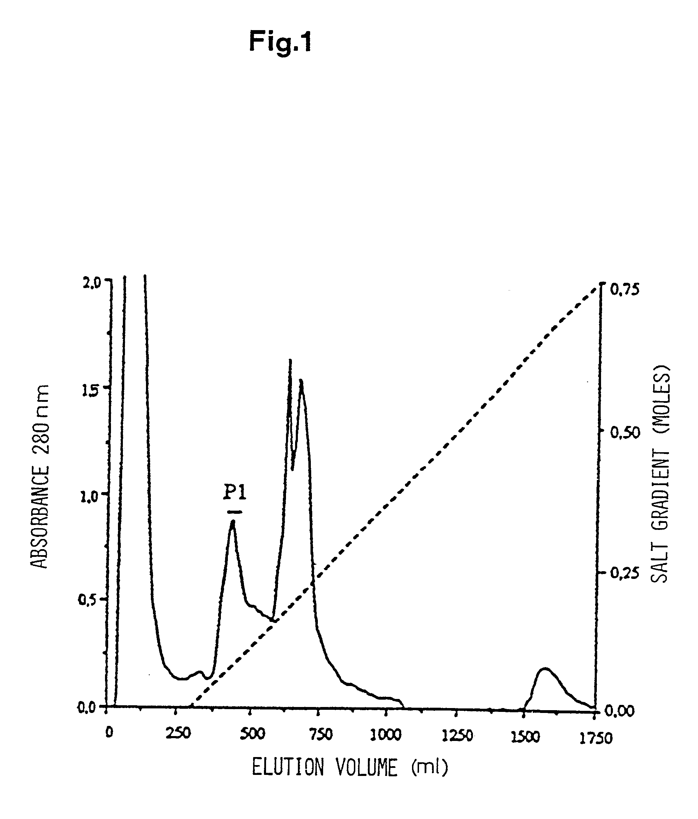

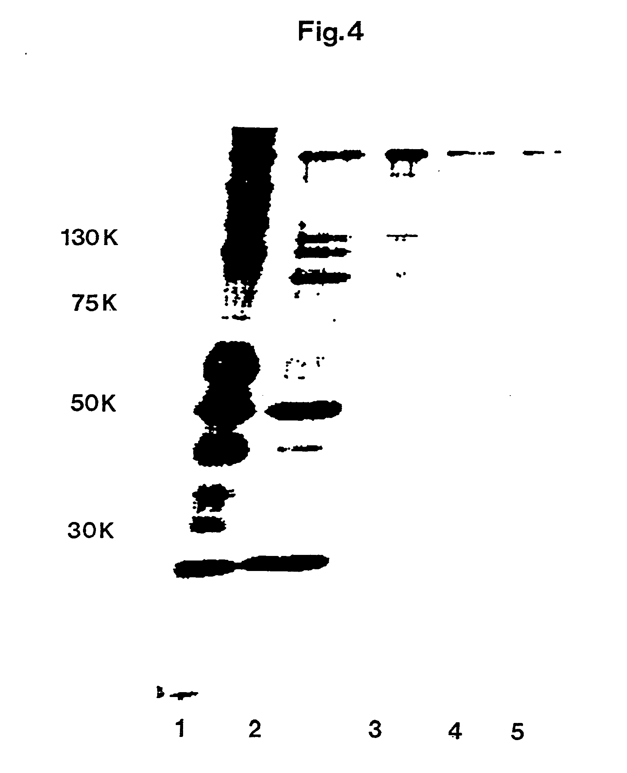

Bovine GBM was extracted with 6 M guanidine HCl, 0.05 M Tris-HCl, pH 7.5 in presence of protease inhibitors to minimize endogenous proteolysis. The extract was initially passed over DEAE Sephadex column equilibrated with 6 M urea, 0.05 M Tris-HCl, pH 8.4. All of this protein bound to the column and could be eluted early, as a broad peak between 0.05-0.20 M NaCl (Fig. 1 & 4) as determined by ELISA and SDS-PAGE.

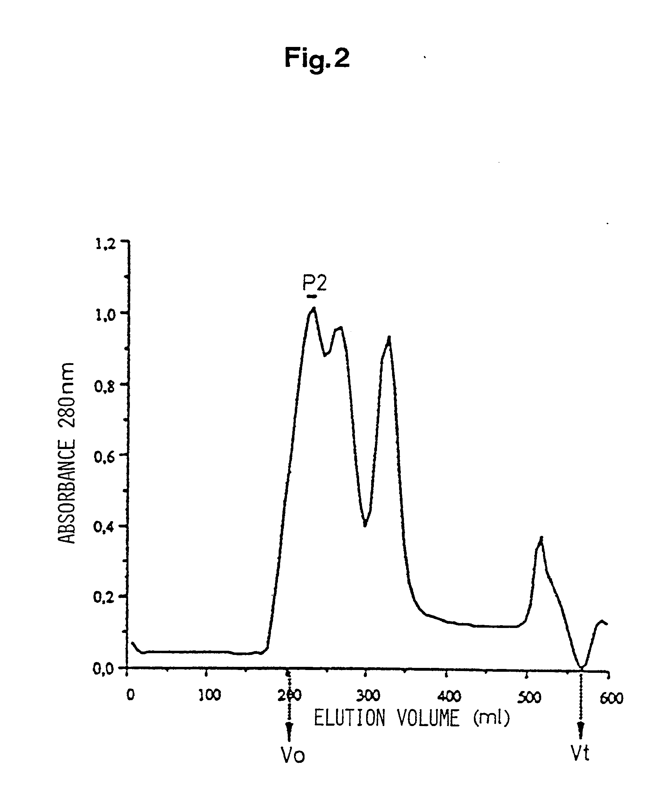

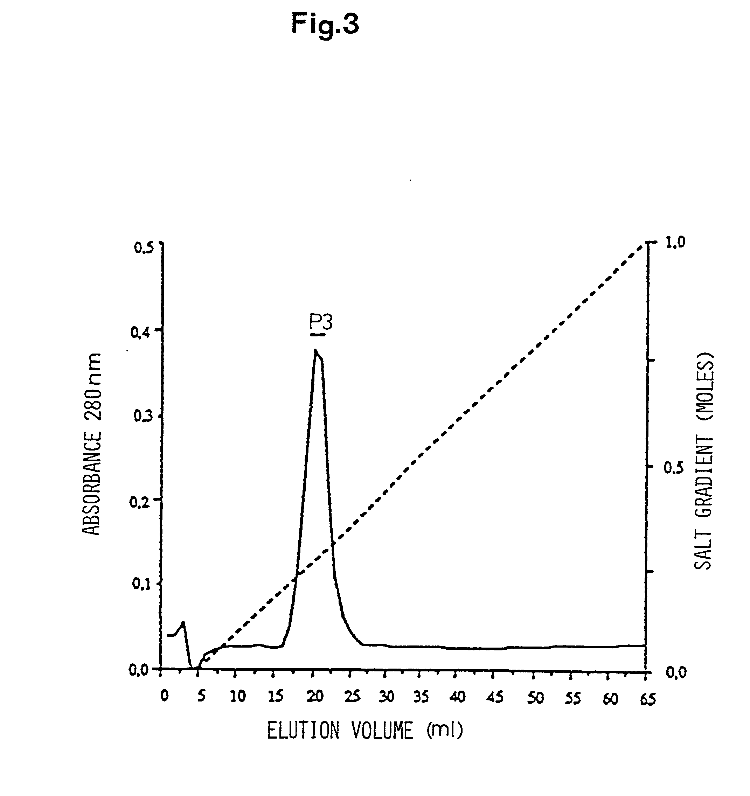

Chromatography on a Sephacryl S-300 column of the fractions containing this protein resulted in separation of its various peptides (Fig. 2 & 4). The protein was further purified by chromatography on a Mono-S FPLC column equilibrated with 6 M urea, 0.02 M sodium acetate, pH 4.8. All of it bound to the column and could be eluted as a sharp peak at 0.22-0.28 M NaCl (Fig. 3 & 4). Final purification of this protein was achieved by rechromatography on a Sephacryl S-300 column. The protein thus obtained was approximately 95% pure electrophoretically.

## EXAMPLE 4

### Specificity of the antibodies to the crude guanidine extract of GBM

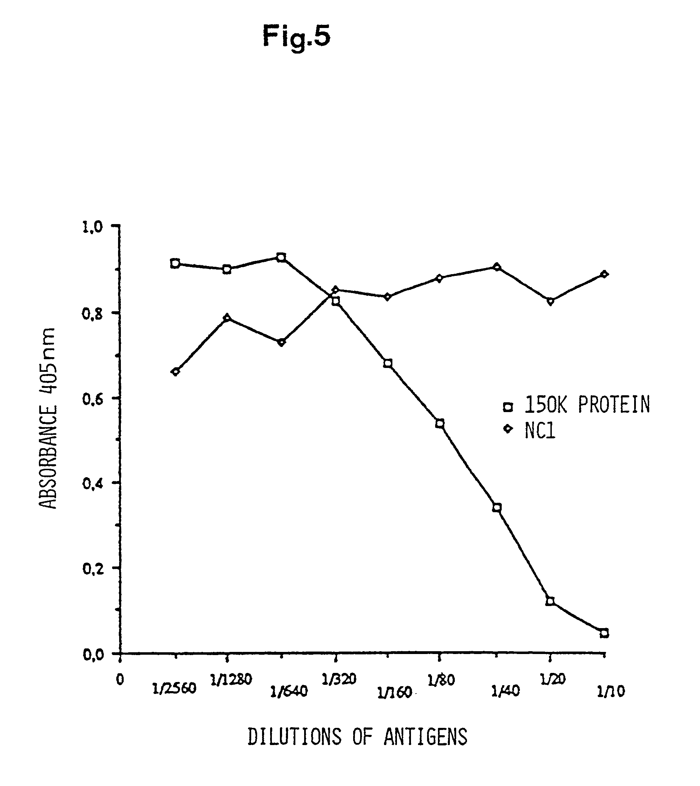

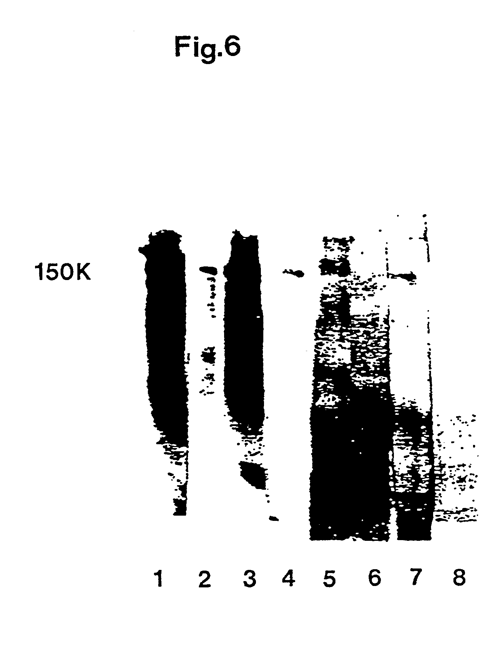

In order to study the specificity of the antibodies to the crude guanidine extract of GBM a study comprising of competitive ELISA was performed. Crude guanidine extract was coated to microtiter plates and a representative serum (Serum that had previously shown antibodies to the 150 K protein), mixed with serial dilutions of 150 K were added and ELISA performed. The activity of the antibodies to the crude extract was well blocked by the 150 K protein (Fig. 5). Furthermore in an immunoblotting experiment, crude guanidine extract of bovine GBM and the 150 K protein were electrophoresed on SDS polyacrylamide slab gel. The proteins were transferred electrophoretically to nitrocellulose sheets and immunostained with a positive serum for antibodies to the 150 K protein (Serum 1) and one negative for those antibodies (Serum 2). In addition, for control, transblotted proteins were immunostained with the reference serum (Rabbit anti-150 K serum). Furthermore, one set was incubated with Serum 1 already mixed with the 150 K protein. It was observed that staining pattern for the reference serum and serum 1 was identical. Both stained the pure entactin band and the corresponding band in the crude guanidine extract No staining was observed with serum 2 or with the mixture of Serum 1 and the 150 K protein (Fig. 6).

## EXAMPLE 5

### Identification of the 150 K Protein as entactin :

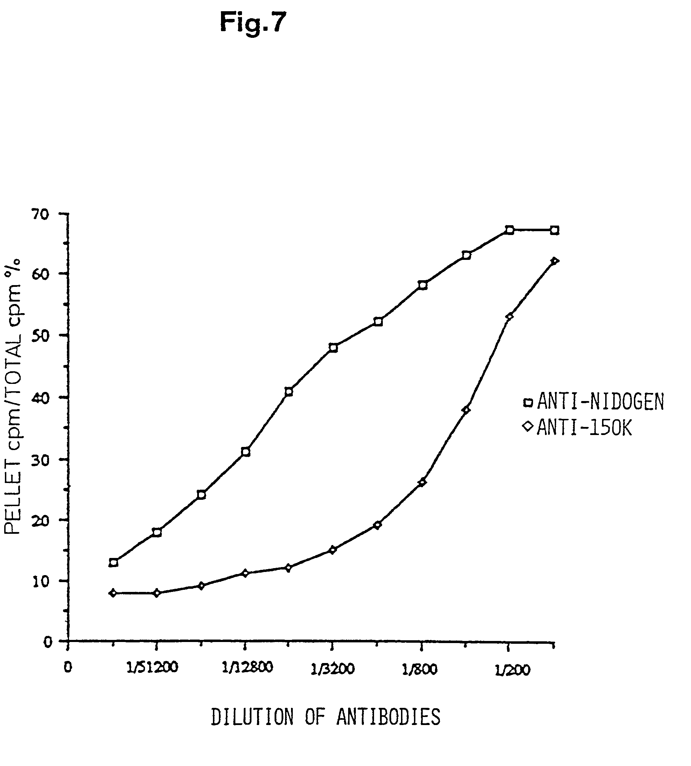

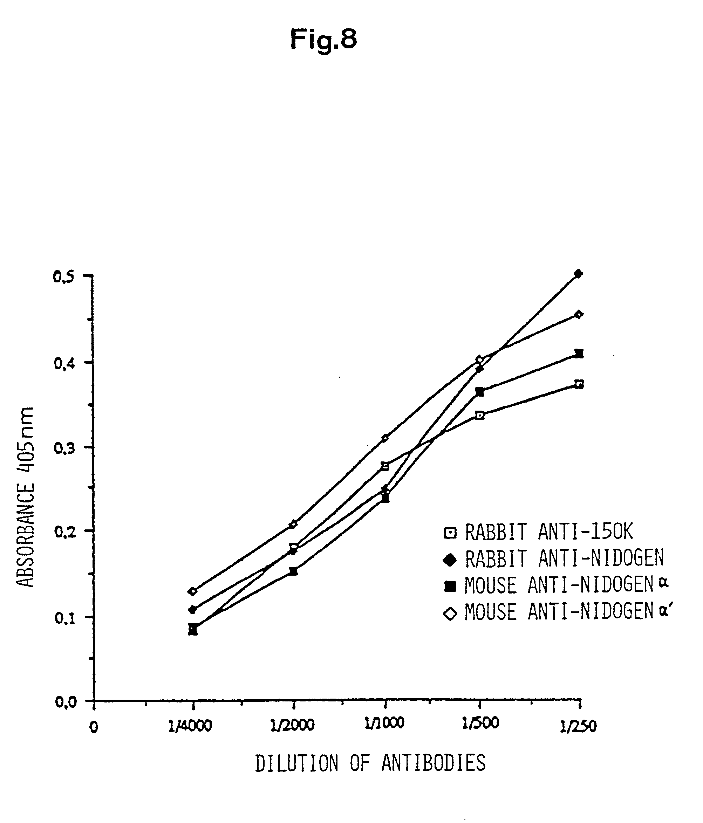

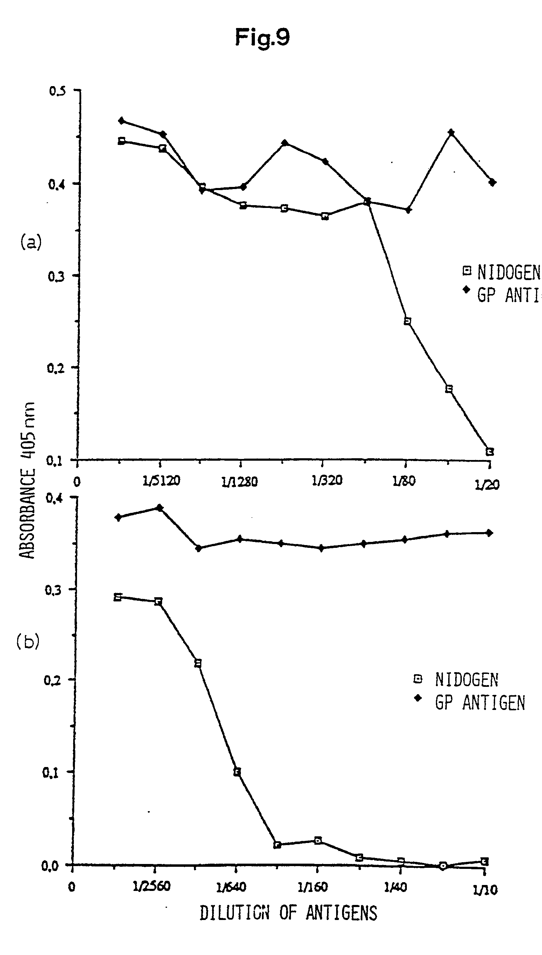

Aminoacid composition of the 150 K protein is summarized in table 1. It is comparable to that of entactin purified by Carlin, et al., 1981 (1) and of nidogen purified by Timpl, et al., 1983 (2) (Table 1). Furthermore, this 150 K protein was radiolabeled with $^{125}$I and precipitated with serial dilutions of two different antibodies (anti-nidogen and anti-150 K antibodies) in a RIA experiment. The amount of the radiolabeled protein precipitated by the two antibodies was similar (Fig. 7). In addition, the reactivity of the 150 K protein to four different antibodies (Rabbit anti-150 K polyclonal, rabbit anti-nidogen polyclonal, and mouse anti-nidogen monoclonal α and α′antibodies) in a sandwich ELISA was comprarable (Fig. 8). Moreover nidogen was able to inhibit the activity of anti-150 K antibodies both in a representative serum and in the rabbit anti-150 K serum in an inhibition ELISA (Fig. 9). Taken together the results of the amino acid analysis, the RIA and the ELISA experiments suggest that the 150 K protein which we isolated from the crude 6 M guanidine-HCl extract of bovine GBM is entactin/nidogen.

## EXAMPLE 6

### Detection of anti-entactin antibodies in patients' sera by ELISA :

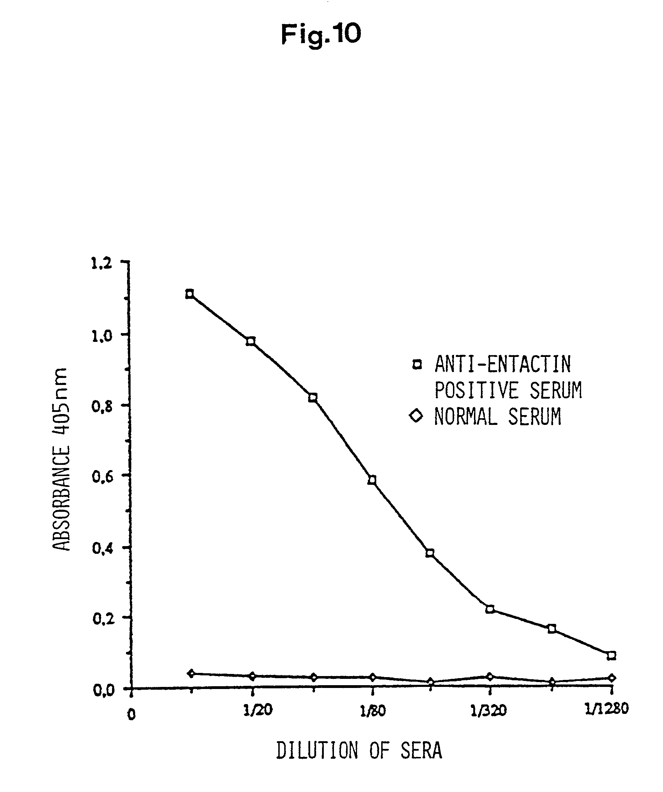

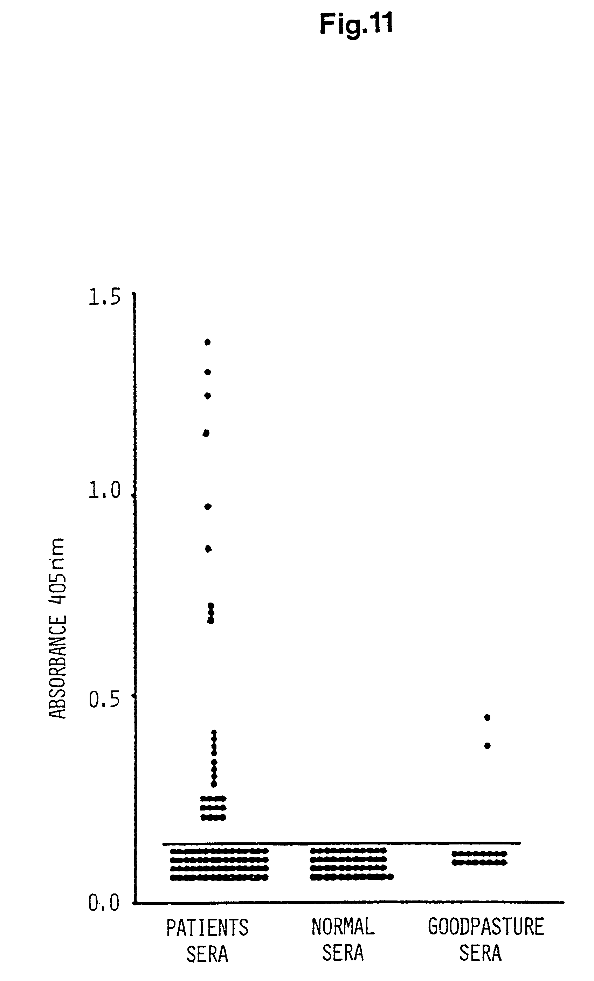

Entactin was able to coat well to the microtiter plates. Moreover the assay was quite sensitive as determined by the dilution curve of a representative serum where the serum could be diluted 1280 times before its absorbance became equal to that of the normal serum (Fig. 10). Additionally, since the absorbance depended on concentration, it indicated a specific binding of immunoglobulins to the coated antigen. Analysis for the presence of anti entactin antibodies, by this ELISA, in the sera from eighty nine patients with renal disease who already had shown IgG/IgM/IgA antibodies to the crude guanidine extract of GBM showed that thirty two patients had positive results. None had IgA antibodies to entactin. None of the forty five normal sera had positive reaction for anti-entactin antibodies while only 2 of the 18 Goodpasture sera analysed showed positive results. (Table 2, Fig. 11).

## EXAMPLE 7

### Role of anti-Gal α 1-3 Gal antibodies :

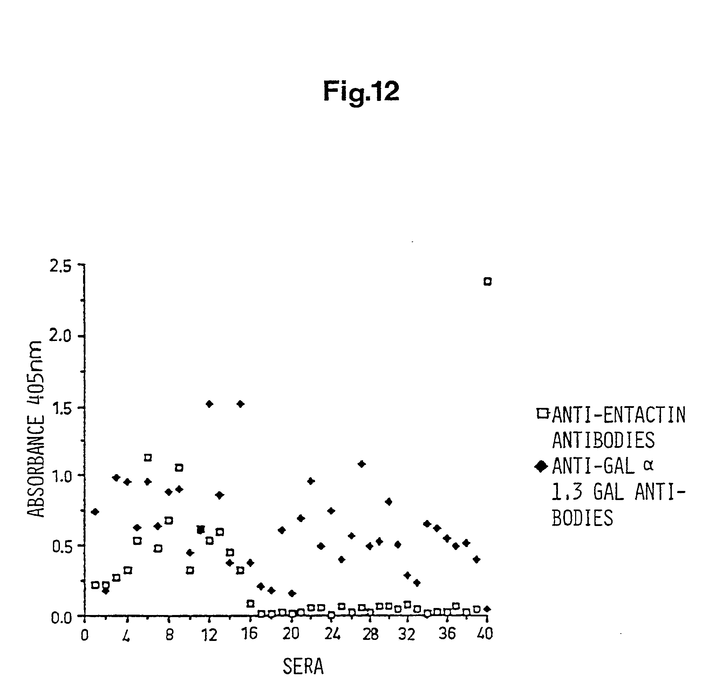

Glycoproteins from bovine origin may contain the carbohydrate epitope, Gal α 1,3 Gal. Since anti-Gal α 1,3 Gal is the most common natural antibody in man (7) it was important to rule out that the antibodies that we measured in patients'sera were anti-entactin antibodies and not anti-Gal α 1,3 Gal antibobies. Hence, we randomly selected 40 different sera including sera from the patients as well as normal sera and simultaneously tested for the presence of the two antibodies. It was observed that most of these sera, including normal sera had high titres for the antibodies to Gal $\alpha$ 1,3 Gal, while only a few had high titres for anti-entactin antibodies (Fig. 12). There was no correlation between the presence of anti-entactin and anti-Gal $\alpha$ 1,3 Gal antibodies (r =+0,185 ; p > 0,05). Furthermore, in a study comprising of competitive ELISA, as described under material and methods, Gal $\alpha$ 1,3 Gal was not able to block anti-entactin antibodies (Data not shown).

In the development of the present invention we have isolated entactin, a sulphated glycoprotein (M.W. 150,000), in genuine form as shown by aminoacid analysis (Table 1), by RIA using two different antibodies (Fig. 7) and by ELISA experiments (Fig. 8 & 9). Furthermore, on coating entactin to microtiter plates and performing ELISA we have shown that many sera that had previously reacted with the crude extract have IgG/IgM antibodies to entactin. Interestingly, none of the normal sera tested showed any reaction to entactin (Fig. 11, Table 2). Futher proof that the antibodies to the crude 6 M guanidine-HCl extract of GBM in these sera were actually directed against entactin was obtained when the activity of antibodies to the crude guanidine extract in a representative serum was blocked by entactin on a competitive ELISA (Fig. 5) and immunoblotting experiments.

Since we isolated entactin from bovine GBM, there was a distinct possibility that it might contain Gal $\alpha$ 1,3 Gal, a carbohydrate epitope present in many mammalian species but lacking in man although almost all human beings posses natural IgG anti-Gal $\alpha$ 1,3 Gal antibodies in their blood (7). Therefore, it was necessary for us to examine whether the antibodies in the sera of various patients positive for entactin were actually directed against entactin and not, as in case of laminin in Chaga's disease (8,9) and American cutaneous Leishmaniasis (9) directed against Gal $\alpha$ 1,3 Gal. It could be observed that both anti-entactin positive and negative sera as well as normal sera showed antibodies to Gal $\alpha$ 1,3 Gal (Fig. 12). There was no correlation between the presence of anti-entactin and anti-Gal $\alpha$ 1,3 Gal antibodies (r = +0,185 ; p > 0,05). Futhermore, Gal $\alpha$ 1,3 Gal was not able to block the antibodies to entactin on competitive ELISA. These observations collectively imply that anti-entactin antibodies that we measured are not the same antibodies which are directed against Gal $\alpha$ 1,3 Gal epitope. In other words anti-entactin and anti-Gal $\alpha$ 1,3 Gal antibodies are two different antibodies.

For the present study we randomly selected sera from various patients with glomerulonephritis who had high IgG/IgM/IgA antibodies titers against crude guanidine extract of GBM. Most of these patients had systemic connective tissue diseases with varying degree of renal involvement. About one third of these patients showed IgG/IgM antibodies to entactin. Only two of the 18 Goodpasture sera showed antibodies to entactin and while one of them had systemic vasculitis and the other had an atypical presentation with positive tests for anti- nuclear antibodies. All these results indicate that there is a distinct group of patients with glomerulonephritis who show auto antibodies to entactin. Most of these patients are generally classified as having immune complex disease on the basis of appearance of granular pattern of antibody deposition along the GBM on immunofluorescence microscopy. Studies in experimental models of autoimmune nephritis such as mercuric chloride induced nephritis in Brown Norway rats and more recently, in murine GvH disease model which is akin to the clinical syndrome of SLE (10) have shown that to begin with the antibodies are deposited linearly along the GBM but gradually the pattern changes to granular type probably because of the reorganization of the antigen antibody complexes. Such phenomenon possibly also exists in human beings but will require early as well as sequential renal biopsies for its elucidation. Nevertheless the observations in experimental models implicate that there is no clear cut demarcation between the immune complex and the anti-GBM glomerulonephritis classified on the basis of granular or linear pattern on immunofluorescence microscopy. Many of the immune complex glomerulonephritis such classified may actually be mediated by anti-GBM antibodies directed to certain auto antigens in the basement membrane as is probably the case with those patients who have circulating antibodies to entactin.

## REFERENCES

1. Marian E. Durkin et al. Amino Acid Sequence and Domain Structure of Entactin ; The Journal of Cell Biology, vol. 107. (1988) 2749-2756.

2. Karlheinz Mann et Al. : Amino Acid sequence of mouse nidogen, a multidomain basement membrane protein with binding activity for laminin, collagen IV and cells ; the EMBO Journal, vol. 8 No. 1, pp 65-72, 1989.

3. Wieslander, J. ; Bygren, P. and Heinegard, D. : Isolation of the specific glomerular basement membrane antigen involted in Goodpasture syndrome. *Proc.Natl. Acad. Sci.*, 81 : 1544-1548, 1984.

4. Laemmli, U.K. : Cleavage of structural proteins during the assembly of the head of bacteriophage T4. *Nature*, 227 : 680-685, 1970.

5. Thorell, J.I. and Larson, S.M. : Radioligands. *In Radioimmunoassay and Related Techniques. Methodology and clinical applications.* Ed. Thorell, J.I. and Larson, S.M. (C.V. Mosby company, St Louis, USA), 1978, pp. 32-49.

6. Burnett, W.N. : "Western blotting" : Electrophoretic transfer of proteins sodium from dodecyl sulphate polyacrylamide gel to unmodified nitrocellulose and radiographic detection with antibody and radioiodinated protein A. *Anal Biochem.,*112 : 195-203, 1981.

7. Galili, U., Shohet, S.B., Kobrin, E., Stuls, C.L.M. and Macher, B.A. : Man, apes, and old world monkeys differ from other mammals in the expression of $\alpha$-galactosyl epitopes on nucleated cells. *J. Biol. Chem.* 263,17755-17762, 1988.

8. Szarfman, A., Terranova, V.P., Rennard, S.I., Foidart, J.M., de Fatima Lima, M., Scheinman, J.I. and Martin, G.R. : Antibodies to laminin in Chaga's disease. *J. Exp. Med.* 155, 1161-1171, 1982.

9. Toowin, H., Rosenfelder, G., Wieslander, J., Avila, J.L., Rojas, M., Szarfman, A., Esser, K., Nowack, H. and Timpl, R. : Circulating antibodies to mouse laminin in Chaga's disease, American cutaneous leishmaniasis, ans normal individuals recognize terminal galactosyl($\alpha$ 1-3)-galactose epitopes. *J. Exp. Med.* 166, 419-432, 1987.

10. Bruijn, J.A., Hogendoorn, P.C.W., Hoedemaeker, Ph.J. and Fleuren, G.J. : The extracellular matrix in pathology. J.Lab.Clin.Med. 111, 140-149, 1988.

11. Engwall and P. Perlmann : Immunochemistry 8, 871 (1971).

## Table 1

*Aminoacid composition of the 150K protein compared to the published aminoacid composition of nidogen/entactin*

| Aminoacid* | 150 K | Nidogen 150 (26) | Entactin (25) |
|---|---|---|---|
| Asp | 100 | 99 | 113 |
| Thr | 64 | 66 | 64 |
| Ser | 98 | 85 | 84 |
| Glu | 118 | 113 | 123 |
| Pro | 72 | 67 | 76 |
| Gly | 104 | 105 | 111 |
| Ala | 73 | 60 | 67 |
| Cys | 23 | 32 | 24 |
| Val | 43 | 57 | 44 |
| Met | 9 | 5 | 10 |
| Ile | 28 | 37 | 27 |
| Leu | 74 | 66 | 69 |
| Tyr | 34 | 36 | 34 |
| Phe | 46 | 36 | 34 |
| His | 36 | 33 | 28 |
| Lys | 20 | 31 | 34 |
| Arg | 58 | 55 | 51 |
| Trp | - | 17 | 7 |

*\* Per 1000 aminoacid residues*

8

## Table 2

*Anti-entactin antibodies in sera from patients with various types of glomerulonephritis*

| Sera | IgM | IgG | IgA | Both IgG/IgM | Total |
|------|-----|-----|-----|--------------|-------|
| Normal n=45 | 0 | 0 | 0 | 0 | 0 |
| GN n=89 | 22 | 7 | 0 | 3 | 32 |
| GP n=18 | 0 | 2 | 0 | 0 | 2 |

GN: Sera from patients with different types of glomerulonephritis who had high titres of IgG/IgM/IgA antibodies to the crude 6M Guanidine-HCl extract of GBM

GP: Sera from patients with Goodpasture Syndrome

Brief description of the annexed drawings

**Fig. 1 :** *DEAE cellulose chromatography of the 6 M guanidine-HCl extract of bovine GBM :* The sample was dialysed against 6 M urea, 0.05 M Tris-HCl, pH 8.4, containing protease inhibitors and applied to a DEAE cellulose column equilibrated with the same buffer. The bound proteins were eluted with a linear gradient (0-0.75 M NaCl). P1 denotes the pool containing the 150 K protein which was used for further purification.

**Fig. 2 :** *Sephracyl S-300 gel filtration chromatography of the 150 K protein :* The pool P1 from the DEAE cellulose chromatography was concentrated and applied to a Sephacyl S-300 column equilibrated with 6 M guanidine-HCl, 0.05 M tris-HCl, pH 7.5 containing protease inhibitor. P2 indicates the pool containing the 150 K protein. The void ($V_0$) and the total ($V_t$) volumes of the column are marked by the arrows.

**FIG. 3 :** *Purification of the 150 K protein on a Mono-S FPLC column :* The pool P2 from the S-300 chromatography was concentrated and dialysed against 6 M urea, 0.02 M Na acetate, pH 4.8 containing protease inhibitors and applied to a Mono-S FPLC column equilibrated with same buffer. The bound proteins were eluted using a linear salt gradient (0-1 M NaCl). P3 indicates the pool containing the 150 K protein.

**FIG. 4 :** *SDS-PAGE of the pool containing 150 K protein :* The fractions after each chromatographic step were subjected to electrophoresis on a polyacrylamide gradient (6-25%) slab gel and stained with Commasie Brilliant blue. All samples were reduced with 1% mercaptoethanol prior to electrophoresis. Lane 1: Starting material (6 M guanidine-HCl extract of bovine GMB), lane 2 : Pool P1 after DEAE chromatography, lane 3 : Pool P2 after S-300 gel filtration chromatography, lane 4 : Pool P3 after Mono-S FPLC, lane 5 : purified 150 K protein after rechromatography of pool P3 on Sephacryl S-300 column.

**FIG. 5 :** *Specificity of the antibodies to the crude guanidine extract of GBM :* Crude guadinide extract of bovine GBM was coated to a microtiter plate and one representative serum (serum that had previously shown antibodies to the 150 K protein), mixed with serial dilutions of the 150 K protein (150 µg/ml undiluted) and NCl (200 µg/ml undiluted) were added and ELISA performed. The 150 K protein was abble to inhibit the antibodies to the crude extract in the representative serum. No inhibition was observed with NCl.

**FIG. 6 :** *Specificity of the antibodies to 6 M guanidine-HCl extract of bovine GBM in an immunoblotting experiment :* Crude guanidine extract and the purified 150 K proteins were electrophoresed on a 3-12.5% polyacrylamide slab gel. The separated proteins were transferred electrophoretically to nitrocellulose papers and immunostained with rabbit anti-150 K serum (reference serum), a serum positive for anti-150 K antibodies (serum 1) a serum negative for those antibodies (serum 2) and a mixture of serum 1 and 150 K protein. Lanes 1 and 2 represent the staining of the crude guanidine extract (CGE) and 150 K protein respectively with the reference serum (S-R) ; lanes 3 and 4, the staining of CGE and 150 K with serum 1; lanes 5 and 6, the staing of CGE and 150 K with serum 2 while lanes 7 and 8 represent the staining of CGE and 150 K prorein with the mixture of serum 1 and 150 K protein.

**FIG. 7** : *Precipitation of the 150 K protein by two different antibodies in a radioimmunoassay :* The 150 K protein was radiolabeled with $^{125}$I and mixed with serial dilutions of two different antibodies (anti-nidogen and anti-150 K antibodies) and RIA performed using double antibody technique. The amount of precipitation of the 150 K protein by two antibodies was comparable.

**FIG. 8** : *Activity of the 150 K protein to differents proteins on a sandwich ELISA :* The isolated 150 K protein was coated to microtiter plates and ELISA performed using 4 different first antibodies (Rabbit anti-150 K, rabbit anti-nidogen, mouse anti-nidogen $\alpha$ and $\alpha'$monoclonal antibodies). The 150 K protein reacted well to the four antibodies

**FIG. 9** : *Inhibition of anti-150 K antibodies by nidogen :* Serial dilutions of nidogen and Goodpasture antigen were mixed with a fixed dilution of a representative serum and rabbit anti-150 K antibodies respectively, transferred to a microtiter plate coated with the 150 K protein and ELISA performed. Nidogen was able to inhibit anti-150 K antibodies both in the representative serum (a) and in the rabbit anti-150 K serum (b), while no inhibition was observed with the Goodparture antigen.

**FIG. 10** : *Sensitivity of ELISA for detection of anti-entactin antibodies :* A microtiter plate was coated with entactin and serial dilutions of a representative serum (serum that had previously shown antibodies to entactin) and a normal serum were added and ELISA performed. The representative serum had to be diluted 1280 times before its optical density became equal to that of the normal serum.

**FIG. 11** : *Anti-entactin antibodies in patients with glomerulonephritis who have high IgG/IgM/IgA antibodies to the crude guanidine extract of GBM :* 89 sera patients with different types of glumerulonephritis, 18 sera from patients with Goodpasture Syndrome and 45 normal were analysed for the presence of anti-entactin antibodies by ELISA. The results are summarized in table 2.

**FIG. 12** : *Correlation between the presence of anti-entactin and anti-gal $\alpha$ 1,3 gal antibodies :* 40 different sera including sera from patients with different types of glomerulonephritis and normal sera were randomly selected for simultaneous estimation of anti-entactin and anti-gal $\alpha$ 1,3 gal antibodies. Most of these sera had high titres of anti-gal $\alpha$ 1,3 gal antibodies while only a few of them had high titres of anti-entactin antibodies. There was no correlation between the presence of the two antibodies in those sera ($r = +0.185$ ; $p > 0.05$).

## Claims

1. A diagnostic process for the detection of renal disorders in mammals including man, comprising the steps:
   a) obtaining a test specimen from the mammal subject to diagnosis ;
   b) reacting entactin or an active fragment thereof with said specimen ; and
   c) registering whether interaction occurs between the entactin and said specimen indicating the presence or absence of said disorder.

2. A diagnostic process according to claim 1, wherein the test specimen used is selected from the group comprising body fluids.

3. A diagnostic process according to claim 2, wherein the test specimen is constituted by a blood sample.

4. A diagnostic process according to any preceding claim, wherein the registration of step c) is performed using ELISA, RIA, hemagglutination-inhibition or the like.

5. A diagnostic process according to any preceding claim for the detection of proliferative glomerulonephritis.

6. A diagnostic process according to any preceding claim, wherein step b) is performed using entactin.

7. Entactin or an active fragment thereof for diagnostic purpose.

8. Entactin or an active fragment thereof according to claim 7 for use in the diagnosis of renal disorders.

9. Entactin or an active fragment thereof according to claim 8 for use in the diagnosis of proliferative glomerulonephritis.

10. Entactin for use in the diagnosis of renal disorders.

11. Entactin for use in the diagnosis of proliferative glomerulonephritis.

12. A diagnostic composition containing entactin or an active fragment thereof in combination with a diagnostically acceptable carrier.

Fig.1

# Fig. 2

# Fig.3

# Fig.4

130 K

75 K

50 K

30 K

1    2       3    4    5

# Fig.5

# Fig.6

150K

1 2 3 4 5 6 7 8

# Fig.7

# Fig.8

# Fig.9

# Fig.10

# Fig.11

# Fig.12

EP 0 430 914 A1

European Patent Office

# EUROPEAN SEARCH REPORT

Application Number

EP 90 85 0381

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 5) |
|---|---|---|---|
| P,X | KIDNEY INTERNATIONAL, vol. 38, no. 2, 1990, pages 263-272; R. SAXENA et al.: "Entactin: A possible auto-antigen in the pathogenesis of non-Goodpasture anti-GBM nephritis" * Abstract; page 264, column 2, lines 37-48 * | 1-11 | G 01 N 33/68 G 01 N 33/564 C 07 K 13/00 |
| D,A | JOURNAL OF CELL BIOLOGY, vol. 107, no. 6, part 2, December 1988, pages 2749-2756, The Rockefeller University Press; M.E. DURKIN et al.: "Amino acid sequence and domain structure of entactin. Homology with epidermal growth factor precursor and low density lipoprotein receptor" * Whole document * | 1 | |
| | | | TECHNICAL FIELDS SEARCHED (Int. Cl.5) G 01 N C 07 K |

The present search report has been drawn up for all claims

| Place of search | Date of completion of the search | Examiner |
|---|---|---|
| THE HAGUE | 13-02-1991 | MERLU B.H.A. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)

24